# EUROPEAN PATENT SPECIFICATION

(11) **EP 3 821 935 B1**
(45) Date of publication and mention of the grant of the patent: **18.06.2025**
(21) Application number: 19860360.7
(22) Date of filing: 04.07.2019
(51) Int. Cl.: A61M 25/09, A61B 18/14, A61B 18/00

(54) **GUIDE WIRE JOINT**
FÜHRUNGSDRAHTGELENK
RACCORD DE FIL DE GUIDAGE

(30) Priority: 11.09.2018 CN 201811060589
(43) Date of publication of application: 19.05.2021
(73) Proprietor: Micro-Tech (Nanjing) Co., Ltd., Nanjing, Jiangsu 210032 (CN)
(72) Inventor: LI, Xiaochun, Nanjing, Jiangsu 210032 (CN); XU, Chaowei, Nanjing, Jiangsu 210032 (CN); LI, Changqing, Nanjing, Jiangsu 210032 (CN); LENG, Derong, Nanjing, Jiangsu 210032 (CN); LIU, Chunjun, Nanjing, Jiangsu (CN)
(74) Representative: Reitstötter Kinzebach
(86) International application number: PCT/CN2019/094692
(87) International publication number: WO 2020/052331

(56) References cited:
- EP-A2- 0 328 760
- EP-A2- 0 328 760
- WO-A2-2005/032638
- CN-A- 1 047 036
- CN-A- 102 512 272
- CN-A- 106 604 698
- CN-A- 109 044 525
- CN-U- 209 301 303
- US-B1- 6 190 333
- US-B1- 6 190 333

## Description

### FIELD OF THE PRESENT DISCLOSURE

The invention relates to the field of a medical device, an in particular to a guide wire joint.

### BACKGROUND OF THE PRESENT DISCLOSURE

Endoscopic sphincterotomy (EST) is a further developed treatment technique based on the diagnostic technique of endoscopic retrograde cholangiopancreatography (ERCP), in which the duodenal papillary sphincter and the end portion of the common bile duct are incised under an endoscope by using a high-frequency electric incision knife.

Clinically, EST is widely used in the treatment of choledocholithiasis, biliary pancreatitis, acute obstructive suppurative cholangitis, periampullary tumor, biliary ascaris, benign stricture of the end of the common bile duct, Oddi sphincter dysfunction, and the like. After the endoscopic papillary sphincterotomy is implemented, combining with lithotripsy, calculus removal, ascaris removal, nasobiliary drainage, stent drainage, etc., the disease can be partially or completely treated.

The incision is mainly performed by inserting an insulating guide wire into a common bile duct, adhering a negative plate of a high-frequency electric generator to the skin of the buttocks of a patient, connecting a connecting joint of a control handle of an incision knife to a corresponding electrode joint of the high-frequency electric generator, and adjusting the electric incision, the electric coagulation power and the mixing proportion thereof. EST can also be performed by using a simple electric incision current, which can reduce the coagulation effect on nearby tissues in the incision process, thereby being beneficial to maintaining a clear endoscopic field.

In conventional ERCP, a general papillotomy knife generally requires retaining the position of a guide wire (4.5 m) while slowly withdrawing the papillotomy knife from the endoscope after the intubation of the pancreaticobiliary system and the sphincterotomy, which requires the cooperation between doctors and nurses, with an exchange distance of about 1.6 to 2 m. After the papillotomy knife is withdrawn from the endoscope, a withdrawal distance of more than 2 m from the guide wire is required outside the body. In the process, the withdrawal distance of the incision knife is long, so that the operation time is long; the proficiency of the cooperation between doctors and nurses is highly required.

At present, most rapid exchange incision knife products on the market are provided with a guide wire insertion hole on the side wall of a guide wire cavity of an outer tube of an incision knife, or after the hole is provided, a slotted guide wire joint is added to serve as a guide wire pre-assembly inlet. Due to the fact that the opening structure of the side wall of the guide wire of the outer tube is limited by the size of the tube, the opening size is small, and the penetrating pre-assembly operation of the guide wire is inconvenient. Because the slotted guide wire joint is not closed, when a J-shaped guide wire is used in the pre-assembly, there is a certain probability that the guide wire can extend out of the slotted position, although the slotted guide wire joint has a certain guiding effect.

Therefore, in order to save the operation time, realize the single-person operation of a doctor, and ensure good guidance of the guide wire penetrating into the guide wire cavity of the incision knife, there is an urgent need for a guide wire joint which can rapidly separate the guide wire from the side wall of the guide wire cavity of the incision knife to the distal end of the papilla incision knife and shorten the exchange distance of the papilla incision knife to withdraw from the endoscope along the guide wire by slotting the side wall of the guide wire cavity and adding the opening and closing function of the guide wire channel on the guide wire joint in cooperation with a guide wire locking a rapid exchange apparatus. An exemplary guide wire joint is disclosed in US 6,190,333 B1.

### SUMMARY OF THE PRESENT DISCLOSURE

The object of the invention is to design a guide wire joint, so that the guide wire has good guiding property when penetrating into a guide wire cavity, and the penetrating efficiency of the guide wire is improved; secondly, a doctor can realize single-person operation when performing ERCP; finally, shortening the exchange distance of the rapid exchange apparatus to withdraw from the endoscope along the guide wire effectively shortens the operation time and the relieves the pain of a patient.

The invention provides a guide wire joint according to claim 1.

According to an embodiment, the medical device is a rapid exchange apparatus and the guide wire joint can be used in cooperation with the rapid exchange apparatus to achieve the rapid separation of the guide wire from the rapid exchange apparatus.

According to an embodiment, sides of the first cover board and a side of the second cover board away from the connecting part are contacted to form a cylinder-like structure, and the distal end diameter of the cylinder-like structure is smaller than that of the proximal end.

According to an embodiment, the guide wire joint further comprises a switch, and the guide wire joint can be opened by changing or pressing the switch to realize guide wire separation.

According to an embodiment, the switch is located on a side of the first cover board away from the connecting part and has an arch structure.

According to an embodiment, the switch is located between the first cover board and the second cover board and comprises a pressure spring.

According an embodiment, the first cover board and the second cover board are formed by plastic injection molding.

According to an embodiment, the connecting part is of a hinge structure.

According to an embodiment, the second cover board has a rough surface on the outer surface thereof.

According to an embodiment, a rapid exchange apparatus for use in cooperation with a guide wire joint includes an outer sheath tube.

According to an embodiment, the outer sheath tube comprises a guide wire cavity with a C-shaped groove formed in a side thereof.

### BRIEF DESCRIPTION OF THE DRAWINGS

Fig. 1 is a schematic view of a right side opening and closing guide wire joint when it is closed.
Fig. 2 is a schematic view of the right side opening and closing guide wire joint shown in Fig. 1 after it is opened.
Fig. 3 is an enlarged view of the schematic view of the right side opening and closing guide wire joint after it is opened as shown in Fig. 2.
Fig. 4 is a cross-sectional view of the right side opening and closing guide wire joint shown in Fig. 1.
Fig. 5 is a schematic view of an upside opening and closing guide wire joint when it is closed.
Fig. 6 is a schematic view showing an open state of the upside opening and closing guide wire joint shown in Fig. 5.
Fig. 7 is an enlarged view of the schematic view of the upside opening and closing guide wire joint after it is opened as shown in Fig. 6.
Fig. 8 is a schematic view of the upside opening and closing guide wire joint shown in Fig. 5 with the second cover board and the connecting part removed.
Fig. 9 is a cross-sectional view of the upside opening and closing guide wire joint shown in Fig. 5.
Fig. 10 is a front view of an outer sheath tube for use in cooperation with a guide wire joint.
Fig. 11 is a cross-sectional view of an outer sheath tube for use in cooperation with the guide wire joint as shown in Fig. 10.

List of reference numerals in the drawings:

| | | | |
|---|---|---|---|
| 1 | first cover board | 2 | second cover board |
| 3 | connecting part | 4 | connecting piece |
| 5 | rough surface | 6 | switch |
| 7 | first cover board side edge | 8 | guide wire |
| 9 | outer sheath tube | 10 | guide wire passage |
| 11 | guide wire cavity | 12 | C-shaped groove |
| 13 | upper half part | 14 | lower half part |
| 15 | pressure spring | 16 | outer tube |
| 17 | triangular opening | | |

### DESCRIPTION OF THE EMBODIMENTS

The technical means adopted by the invention for achieving the purpose preset for the invention is further described with reference to the following drawings and preferred embodiments of the invention.

The present disclosure provides a guide wire joint which comprises a first cover board, a second cover board, a connecting part for connecting the first cover board and the second cover board, and a connecting piece; the connecting piece can connect the guide wire joint to a medical device; a side of the first cover board and a side of the second cover board that are away from the connecting part are in a contact state or a separated state to adjust closing and opening of the guide wire joint; when in the contact state, a guide wire passage is formed between the first cover board and the second cover board so that a guide wire can be allowed to pass through, and the guide wire joint is in a closed state; when in the separated state, the guide wire may be detached from the guide wire joint, with the guide wire joint in an open state.

According to the guide wire joint provided by the present disclosure, the medical device is a rapid exchange apparatus. The guide wire joint can be used in cooperation with the rapid exchange apparatus to realize the rapid separation of the guide wire and the rapid exchange apparatus, and the specific rapid exchange apparatus can be a papilla incision knife, a stone removing balloon, a radiography tube, a stone removing basket, an expansion balloon, and the like.

According to the guide wire joint provided by the present disclosure, a side of the first cover board and a side of the second cover board that are away from the connecting part are contacted to form a cylinder-like structure, and the distal end diameter of the cylinder-like structure is smaller than that of the proximal end.

According to the guide wire joint provided by the present disclosure, the guide wire joint further comprises a switch, and the guide wire joint can be opened by changing or pressing the switch to realize guide wire separation.

Preferably, the switch is located on a side of the first cover board away from the connecting part and has an arch structure. The switch may also be located between the first cover board and the second cover board, including at least one pressure spring.

According to the guide wire joint provided by the present disclosure, the first cover board and the second cover board are formed by plastic injection molding.

According to the guide wire joint provided by the present disclosure, the connecting part is a hinge structure.

According to the guide wire joint provided by the present disclosure, a rough surface is arranged on the outer surface of the second cover board. The rough surface is a finger clamping position during use such that the friction resistance is increased while the clamping falling off during the opening of the guide wire joint or the separation of the guide wire can be prevented.

According to the guide wire joint provided by the present disclosure, the rapid exchange apparatus for use in cooperation with the guide wire joint includes an outer sheath tube.

Preferably, the outer sheath tube includes a guide wire cavity with a C-shaped groove on a side so that the guide wire can penetrate into the guide wire cavity of the outer sheath tube from the guide wire passage of the guide wire joint.

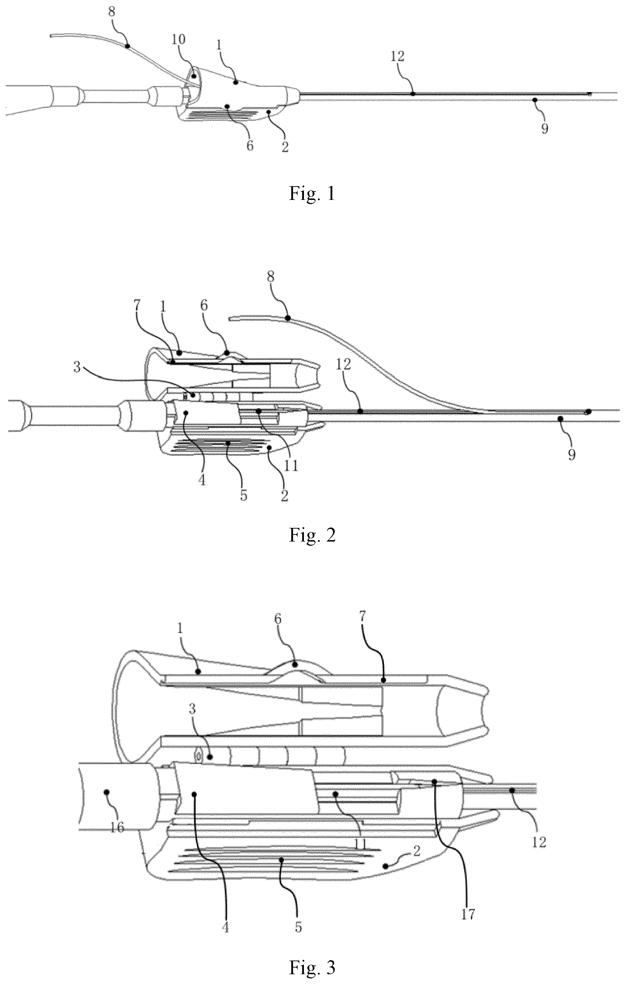

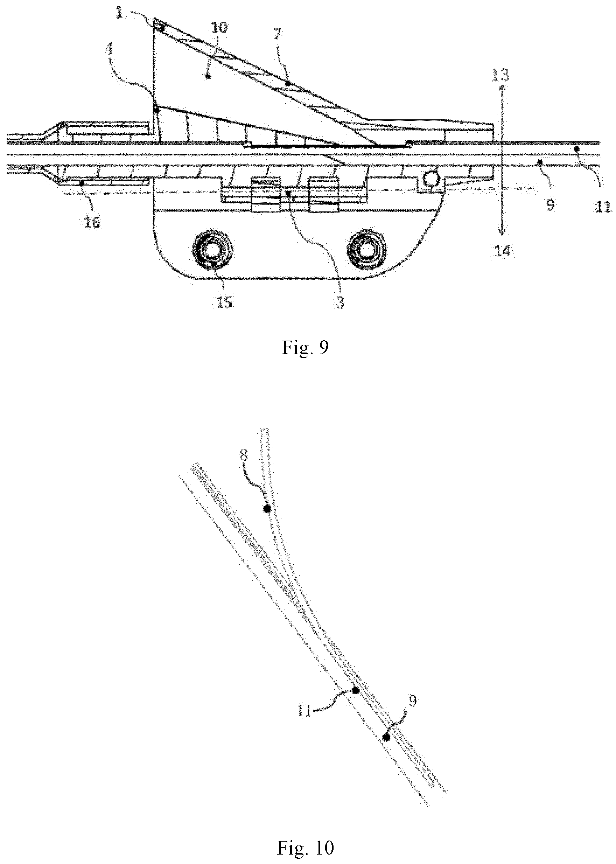

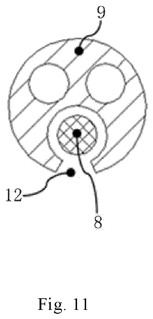

Figs. 1 to 9 illustrate a guide wire joint according to an embodiment of the present invention. Figs. 10 to 11 illustrate an outer sheath tube structure.

In a non-limiting embodiment of the present invention, a guide wire joint is as shown in FIGS. 1-9, including a first cover board 1, a second cover board 2, a connecting part 3 connecting the first cover board 1 and the second cover board 2, and a connecting piece 4. A side of the first cover board 1 and a side of the second cover board 2 that are away from the connecting part 3 may be contacted to form a cylinder-like structure. At the moment, the guide wire joint is in a closed state, a guide wire passage 10 is formed between the first cover board 1 and the second cover board 2 of the guide wire joint, and a guide wire 8 enters the guide wire joint from the proximal end of the guide wire joint through the guide wire passage 10; the distal end diameter of the cylinder-like structure is smaller than that of the proximal end, a side of the first cover board 1 and a side of the second cover board 2 that are away from the shaft tube 3 can be separated to form an opening, at the time the guide wire joint is in an open state, and the guide wire 8 is separated from the guide wire joint from the opening.

The connecting piece 4 is arranged on the second cover board 2 and fixedly connected with the second cover board 2; the connecting piece 4 can also be formed by protruding the inner sides of the first cover board 1 and the second cover board 2 outwards, so that when the guide wire joint is closed, the protrusions on the inner sides of the two cover boards are in contact to form a complete connecting piece 4. The connecting piece 4 can be connected with the rapid exchange apparatus with the thickness from the proximal end of the guide wire joint to the distal end of the guide wire joint getting gradually reduced. The connecting piece 4 can form one guiding channel with the first cover board 1 while being connected to the rapid exchange apparatus to guide the guide wire 8 .

A rough surface 5 is arranged on the outer surface of the second cover board 2, and the rough surface 5 can be a point-shaped protrusion, a stripe-shaped inner concave, and the like. The rough surface is a finger clamping position during use, such that the friction resistance is increased while the clamping falling off during the opening of the guide wire joint or the separation of the guide wire 8 can be prevented.

The guide wire joint can be connected with the outer tube 16 of the rapid exchange apparatus through the connecting piece 4, so that the guide wire 8 penetrates through the guide wire passage 10 of the guide wire joint and extends to the distal end, and finally extends out from the distal end of the outer tube 16. The distal end of the outer tube 16 is provided with an opening to facilitate the rapid separation of the guide wire 8 from the guide wire joint when the guide wire 8 is separated from the rapid exchange apparatus, and preferably, the opening arranged at the distal end of the outer tube 16 is a triangular opening 17. When the guide wire 8 enters the guide wire joint from the guide wire passage 10, the guide wire 8 is guided to rapidly enter the cavity of the rapid exchange apparatus and extends out of the distal end outlet of the rapid exchange apparatus to play a guiding role. When the guide wire 8 is retained within the guide wire cavity 11, the distal end of the triangular opening 17 contacts to retain the guide wire 8 within the guide wire cavity 11. When the guide wire 8 is detached from the rapid exchange apparatus, pulled by a certain force, the guide wire 8 is caused to penetrate through the distal end of the triangular opening 17, thereby separating the guide wire 8 from the guide wire joint.

In particular, the outer tube 16 can be designed to be connected to the outer sheath tube 9, wherein one side of the outer sheath tube 9 is provided with a C-shaped groove 12 so that the guide wire 8 can penetrate from the guide wire passage 10 of the guide wire joint into the guide wire cavity 11 of the outer sheath tube 9. The outer diameter of the outer sheath tube 9 can be designed to be equal to or slightly smaller than the inner diameter of the distal end of the guide wire joint, so that when the guide wire joint is closed, the guide wire 8 can be smoothly inserted into the guide wire cavity 11 of the outer sheath tube 9 from the guide wire passage 10 of the guide wire joint in a better way, reaches the distal end of the rapid exchange apparatus along the guide wire cavity 11, and extends out from the distal end outlet of the rapid exchange apparatus. So that the effect of accurately guiding the advancing direction of the guide wire 8 is achieved and the penetration efficiency of the guide wire 8 is effectively improved.

The connecting part 3 in the guide wire joint can be a hinge structure to fixedly connect the first cover board 1 to the second cover board 2.

The guide wire joint also includes a switch 6 that opens the guide wire passage 10 by changing or pressing to form one opening. When the guide wire joint is opened, the guide wire 8 is separated from the opening of the guide wire joint and the rapid exchange apparatus, thereby achieving rapid exchange.

The switch 6 can be located on one side edge 7 of the first cover board 1 away from the connecting part 3, and has an arch structure. Specifically, it arches out towards the outer side of the guide wire joint to form an arc surface. When the guide wire joint is opened, by changing the switch 6, the side edge 7 of the first cover board is gradually moved away from the second cover board 2 to form an opening, and the guide wire 8 arranged in the guide wire joint is separated from the guide wire joint from the opening.

The switch 6 can also be located between the first cover board 1 and the second cover board 2 and comprises at least one pressure spring 15. The first cover board 1 of the guide wire joint can be opened to form an opening by pressing the second cover board 2 of the guide wire joint, and the guide wire 8 arranged in the guide wire joint is released from the opening.

Therefore, the guide wire joint of the invention is simple and convenient to operate. The guide wire joint can be opened only by changing or pressing the switch 6, and the guide wire 8 is thus separated from the guide wire joint, so that the guide wire 8 is rapidly separated from the rapid exchange apparatus to the far end portion of the rapid exchange apparatus. It is suitable for the rapid exchange process of the rapid exchange apparatus, comprising a papilla incision knife, a stone removing balloon, a radiography tube, a stone removing basket, an expansion balloon, and the like. In addition, the guide wire joint of the invention effectively shortens the exchange distance of the rapid exchange apparatus for withdrawing from the endoscope along the guide wire 8, realizing the rapid withdrawal of the rapid exchange apparatus and effectively shortening the operation time.

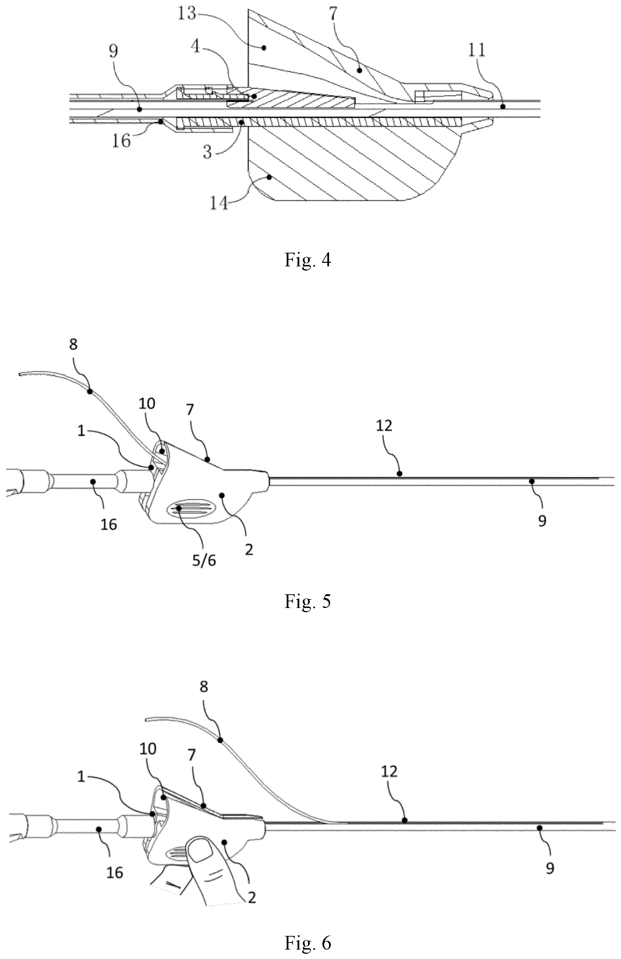

In a non-limiting embodiment of the present invention, as shown in Figs. 1 and 5 which are schematic views of a guide wire joint when it is closed, when the guide wire joint is closed, a side of the first cover board 1 and a side of the second cover board 2 that are away from the connecting part 3 are contacted to form a cylinder-like structure. The guide wire 8 penetrates through the interior of the cylinder-like structure. The distal ends of the first cover board 1 and the second cover board 2 are gradually closed towards the connecting part 3, so that the distal end diameter of the cylinder-like structure is smaller than the proximal end diameter.

As shown in Figs. 1 to 4, the first cover board 1 is an arch structure with a large proximal end diameter and a small distal end diameter. As shown in Fig. 3, the diameter of the first cover board 1 is firstly reduced, then unchanged and finally reduced from the proximal end to the distal end. An angled structure with a certain radian is formed at the joint of the proximal end surface of the second cover board 2 and the side wall of the second cover board 2, and the diameter of the second cover board 2 is firstly unchanged and then reduced from the proximal end to the distal end. The initial positions where the diameters of the first cover board 1 and the second cover board 2 are reduced coincide. Because the distal end diameter of the cylinder-like structure is smaller than the proximal end diameter, when the guide wire joint is closed, the guide wire 8 can be more conveniently transmitted to the distal end of the rapid exchange apparatus after entering the guide wire joint from the guide wire passage 10. As one side of the outer sheath tube 9 of the rapid exchange apparatus is provided with a C-shaped groove structure, the guide wire 8 can be smoothly inserted into the guide wire cavity 11 of the outer sheath tube 9 from the guide wire passage 10 of the guide wire joint so as to enter the distal end of the rapid exchange apparatus, and the exchange distance between the guide wire 8 and the rapid exchange apparatus can be shortened.

A switch 6 is arranged on one side 7 of the first cover board 1 of the guide wire joint away from the connecting part 3, and the switch 6 is arranged on the side edge 7 of the first cover board, and the outer side of the guide wire joint is arched to form an arc surface. When the guide wire joint is opened, by changing the switch 6, the side edge 7 of the first cover board is gradually moved away from the second cover board 2 to form an opening, and the guide wire 8 provided in the guide wire joint is released from the opening.

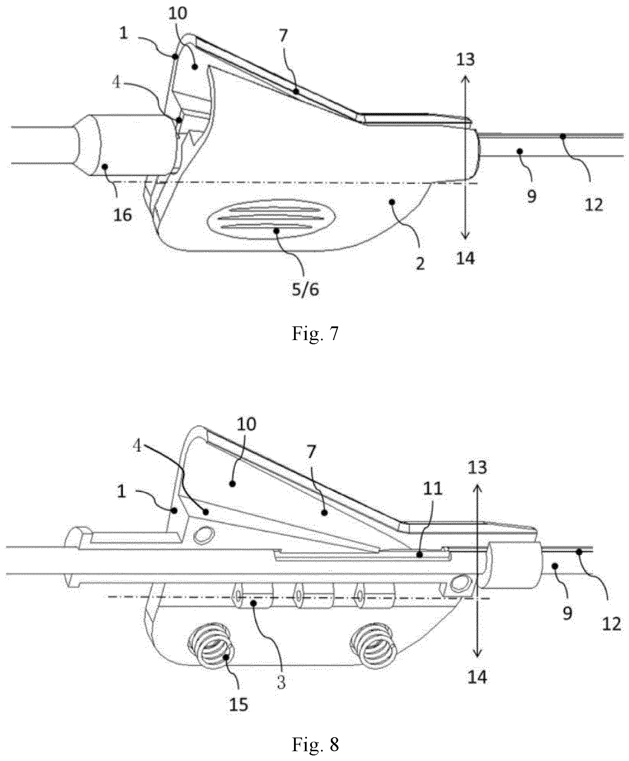

In another embodiment of the present invention, as shown in Figs. 5 to 9, the first cover board 1 and the second cover board 2 are symmetrical structures. As shown in Figs. 8 to 9, the connecting part 3 is located in the middle of the first cover board 1 to divide the first cover board 1 into a curved upper half part 13 and a planar lower half part 14. The upper half part 13 is an arch structure with a large proximal end diameter and a small distal end diameter. The upper half part 13, from proximal end to distal end, decreases in diameter first, then does not change, and finally decreases. The lower half part 14 has one planar structure with two symmetrically distributed grooves for arranging a pressure spring 15. When the second cover board 2 and the first cover board 1 which are symmetrical to the first cover board 1 are combined in the guide wire joint, a cylinder-like structure is formed, and the guide wire joint is in a closed state.

As shown in Figs. 5 to 9, the switch 6 in the guide wire joint is located between the first cover board 1 and the second cover board 2 and comprises at least one pressure spring 15, preferably two pressure springs 15 symmetrically distributed between the lower half parts 14 of the first cover board 1 and the second cover board 2 of the guide wire. By pressing the second cover board 2 of the guide wire joint, the distance between the lower half parts 14 of the two cover boards is reduced. Because the first cover board 1 and the second cover board 2 are connected by the connecting part 3 provided between the upper half parts 13 and the lower half parts 14, when the distance between the two lower half parts 14 is reduced, the upper half parts 13 tend to move away from each other, so that the guide wire joint opens to form an opening, and the guide wire 8 provided in the guide wire joint is released from the opening.

Referring to Fig. 7, which shows the connecting piece 4 in the guide wire joint, wherein the proximal end tip of which terminates at the proximal end tips of the first cover board 1 and the second cover board 2, in which case the guide wire joint is clamped on the outer sheath tube 9 or the outer tube 16. The connecting piece 4 may also be shown in Figs. 8 and 9, wherein the proximal end tip of which is located outside the proximal ends of the first cover board 1 and the second cover board 2, and the outwardly extending portions are fixedly connected to the outer sheath tube 9 or the outer tube 16.

The above are only the preferred embodiments of the present invention, and do not limit the present invention in any form. The scope of the invention is defined by the appended claims.

## Claims

1. A guide wire joint, comprising a first cover board (1), a second cover board (2), a connecting part (3) for connecting the first cover board (1) and the second cover board (2), and a connecting piece (4) for connecting the guide wire joint to a medical device; wherein the one side of the first cover board (1) and the one side of the second cover board (2) are connected together via the connecting part (3), while the other side of the first cover board (1) and the other side of the second cover board (2) are not connected, so that when the other side of the first cover board (1) and the other side of the second cover board (2) are in a contact state a closing of the guide wire joint is realized, and wherein when the other side of the first cover board and the other side of the second cover board are in a separated state, an opening of the guide wire joint is realized; wherein, when in the contact state, a guide wire passage (10) is formed between the first cover board (1) and the connecting piece (4) so that a guide wire (8) can be allowed to enter the guide wire joint through the guide wire passage (10) and penetrate into a guide wire cavity of the medical device, and wherein when in the separated state, said other side of the first cover board (1) and said other side of the second cover board (2) are separated to form an opening, such that the guide wire (8) may be detached from the guide wire joint from the opening in a state where the medical device is not separated from the guide wire joint.

2. The guide wire joint of claim 1, wherein the medical device is a rapid exchange apparatus.

3. The guide wire joint of claim 1, wherein a distal end diameter of a structure formed by contacting sides of the first cover board and the second cover board away from the connecting part is smaller than a proximal end diameter of the structure.

4. The guide wire joint of claim 1, wherein the guide wire joint further comprises a switch (6) for opening the guide wire joint by changing or pressing the switch to separate the guide wire.

5. The guide wire joint of claim 4, wherein that the switch is located on a side of the first cover board away from the connecting part and has an arch structure.

6. The guide wire joint of claim 4, wherein the switch is located between the first cover board and the second cover board and comprises a pressure spring (15).

7. The guide wire joint of claim 1, wherein the first cover board and the second cover board are formed by plastic injection molding.

8. The guide wire joint of claim 1, wherein the connecting part is a hinge structure.

9. The guide wire joint of claim 1, wherein the second cover board has a rough surface (5) on an outer surface thereof.

10. The guide wire joint of claim 2, wherein the rapid exchange apparatus configured for cooperation with the guide wire joint comprises an outer sheath tube.

11. The guide wire joint of claim 10, wherein the outer sheath tube comprises a guide wire cavity with a C-shaped groove formed in a side thereof.

## Patentansprüche

1. Führungsdrahtgelenk, umfassend eine erste Abdeckung (1), eine zweite Abdeckung (2), ein Verbindungsteil (3) zum Verbinden der ersten Abdeckung (1) und der zweiten Abdeckung (2),
und ein Verbindungsstück (4) zum Verbinden des Führungsdrahtgelenks mit einem medizinischen Gerät; wobei die eine Seite der ersten Abdeckung (1) und die eine Seite der zweiten Abdeckung (2) über das Verbindungsteil (3) miteinander verbunden sind, während die andere Seite der ersten Abdeckung (1) und die andere Seite der zweiten Abdeckung (2) nicht miteinander verbunden sind, so dass, wenn die andere Seite der ersten Abdeckung (1) und die andere Seite der zweiten Abdeckung (2) in Kontakt sind, ein Schließen des Führungsdrahtgelenks bewirkt wird, und wobei, wenn die andere Seite der ersten Abdeckung und die andere Seite der zweiten Abdeckung voneinander getrennt sind, ein Öffnen des Führungsdrahtgelenks bewirkt wird;
wobei im Kontaktzustand ein Führungsdrahtdurchgang (10) zwischen der ersten Abdeckung (1) und dem Verbindungsstück (4) gebildet wird, so dass ein Führungsdraht (8) in das Führungsdrahtgelenk durch den Führungsdrahtdurchgang (10) eingeführt werden kann und in einen Führungsdrahthohlraum des medizinischen Geräts eintreten kann, und
wobei, wenn die andere Seite der ersten Abdeckung (1) und die andere Seite der zweiten Abdeckung (2) voneinander getrennt sind, um eine Öffnung zu bilden, der Führungsdraht (8) aus der Öffnung vom Führungsdrahtgelenk gelöst werden kann, wobei das medizinische Gerät nicht von dem Führungsdrahtgelenk getrennt ist.

2. Führungsdrahtgelenk nach Anspruch 1, wobei das medizinische Gerät eine Schnellwechselvorrichtung ist.

3. Führungsdrahtgelenk nach Anspruch 1, wobei ein distaler Enddurchmesser der Struktur, die durch die Kontaktseiten der ersten Abdeckung und der zweiten Abdeckung jenseits des Verbindungsteils gebildet wird, kleiner ist als der proximale Enddurchmesser der Struktur.

4. Führungsdrahtgelenk nach Anspruch 1, wobei das Führungsdrahtgelenk ferner einen Schalter (6) zum Öffnen des Führungsdrahtgelenks durch Umschalten oder Drücken des Schalters umfasst, um den Führungsdraht zu separieren.

5. Führungsdrahtgelenk nach Anspruch 4, wobei sich der Schalter auf einer dem Verbindungsteil abgewandten Seite der ersten Abdeckung befindet und eine bogenförmige Struktur aufweist.

6. Führungsdrahtgelenk nach Anspruch 4, wobei der Schalter zwischen der ersten Abdeckung und der zweiten Abdeckung angeordnet ist und eine Druckfeder (15) umfasst.

7. Führungsdrahtgelenk nach Anspruch 1, wobei die erste Abdeckung und die zweite Abdeckung durch Kunststoffspritzgießen hergestellt werden.

8. Führungsdrahtgelenk nach Anspruch 1, wobei das Verbindungsteil eine Scharnierstruktur ist.

9. Führungsdrahtgelenk nach Anspruch 1, wobei die zweite Abdeckung an ihrer Außenfläche eine raue Oberfläche (5) aufweist.

10. Führungsdrahtgelenk nach Anspruch 2, wobei die Schnellwechselvorrichtung, welche für das Zusammenspiel mit dem Führungsdrahtgelenk konfiguriert ist, ein äußeres Hüllrohr umfasst.

11. Führungsdrahtgelenk nach Anspruch 10, wobei das äußere Hüllrohr einen Führungsdrahthohlraum mit einer in einer Seite davon ausgebildeten C-förmigen Nut umfasst.

## Revendications

1. Raccord de fil de guidage, comprenant un premier panneau de recouvrement (1), un second panneau de recouvrement (2), une partie de liaison (3) pour relier le premier panneau de recouvrement (1) et le second panneau de recouvrement (2), et une pièce de liaison (4) pour relier le raccord de fil de guidage à un dispositif médical ; dans lequel le premier côté du premier panneau de recouvrement (1) et le premier côté du second panneau de recouvrement (2) sont reliés ensemble via la partie de liaison (3), tandis que l'autre côté du premier panneau de recouvrement (1) et l'autre côté du second panneau de recouvrement (2) ne sont pas reliés, de sorte que lorsque l'autre côté du premier panneau de recouvrement (1) et l'autre côté du second panneau de recouvrement (2) sont dans un état de contact, une fermeture du raccord de fil de guidage est réalisée, et dans lequel lorsque l'autre côté du premier panneau de recouvrement et l'autre côté du second panneau de recouvrement sont dans un état séparé, une ouverture du raccord de fil de guidage est réalisée ;
dans lequel lorsqu'il est dans l'état de contact, un passage de fil de guidage (10) est formé entre le premier panneau de recouvrement (1) et la pièce de liaison (4) de sorte qu'un fil de guidage (8) peut être autorisé à entrer dans le raccord de fil de guidage à travers le passage de fil de guidage (10) et à pénétrer dans une cavité de fil de guidage du dispositif médical, et
dans lequel lorsqu'il est dans l'état séparé, ledit autre côté du premier panneau de recouvrement (1) et ledit autre côté du second panneau de recouvrement (2) sont séparés pour former une ouverture, de telle sorte que le fil de guidage (8) peut être détaché du raccord de fil de guidage à partir de l'ouverture dans un état dans lequel le dispositif médical n'est pas séparé du raccord de fil de guidage.

2. Raccord de fil de guidage selon la revendication 1, dans lequel le dispositif médical est un appareil à échange rapide.

3. Raccord de fil de guidage selon la revendication 1, dans lequel un diamètre d'extrémité distale d'une structure formée en mettant en contact des côtés du premier panneau de recouvrement et du second panneau de recouvrement à l'écart de la partie de liaison est inférieur à un diamètre d'extrémité proximale de la structure.

4. Raccord de fil de guidage selon la revendication 1, dans lequel le raccord de fil de guidage comprend en outre un commutateur (6) pour ouvrir le raccord de fil de guidage en modifiant ou en appuyant sur le commutateur pour séparer le fil de guidage.

5. Raccord de fil de guidage selon la revendication 4, dans lequel le commutateur est situé sur un côté du premier panneau de recouvrement éloigné de la partie de liaison et présente une structure arquée.

6. Raccord de fil de guidage selon la revendication 4, dans lequel le commutateur est situé entre le premier panneau de recouvrement et le second panneau de recouvrement et comprend un ressort de pression (15).

7. Raccord de fil de guidage selon la revendication 1, dans lequel le premier panneau de recouvrement et le second panneau de recouvrement sont formés par moulage par injection de plastique.

8. Raccord de fil de guidage selon la revendication 1, dans lequel la partie de liaison est une structure de charnière.

9. Raccord de fil de guidage selon la revendication 1, dans lequel le second panneau de recouvrement présente une surface rugueuse (5) sur une surface extérieure de celui-ci.

10. Raccord de fil de guidage selon la revendication 2, dans lequel l'appareil à échange rapide configuré pour coopérer avec le raccord de fil de guidage comprend un tube de gaine externe.

11. Raccord de fil de guidage selon la revendication 10, dans lequel le tube de gaine externe comprend une cavité de fil de guidage avec une rainure en forme de C formée dans un côté de celle-ci.
